# EUROPEAN PATENT APPLICATION

(11) **EP 4 246 445 A1**
(43) Date of publication of application: **20.09.2023**
(21) Application number: 22166805.6
(22) Date of filing: 05.04.2022
(51) Int. Cl.: G06T 11/00, G06T 13/00, G06V 40/20, G06T 7/70

(54) **ANONYMIZATION OF SUBJECT VIDEO FEEDS**

(30) Priority: 18.03.2022 US 202263321364 P
(71) Applicant: Koninklijke Philips N.V., 5656 AG Eindhoven (NL)
(72) Inventor: CHADUVULA, Siva Chaitanya, Eindhoven (NL); AMTHOR, Thomas Erik, Eindhoven (NL); STAROBINETS, Olga, Eindhoven (NL); FINDEKLEE, Christian, Eindhoven (NL); KOKER, Ekin, Eindhoven (NL); VAN OMMERING, Robert Christiaan, Eindhoven (NL); TELLIS, Ranjith Naveen, Eindhoven (NL); DALAL, Sandeep Madhukar, Eindhoven (NL); Qian, Yuechen, Eindoven (NL)
(74) Representative: Philips Intellectual Property & Standards

(57) **Abstract**

Disclosed herein is a medical system (100, 400) comprising a memory (138) storing machine executable instructions (140), a body pose determination module (146), a character mapping module (154), and a room view generator module (150). The medical system further comprises a camera system (122) configured for acquiring a video stream (142) of a subject support (120) of a medical imaging device (102) within an examination room. The execution of the execution of machine executable instructions causes the computational system to repeatedly: control (200) the camera system to acquire the video stream; sequentially (202) select the individual frame from the video stream; receive (204) the set of pose landmarks for the at least one subject by inputting the individual frame into the body pose determination module; receive (206) the animated room view of the of the medical imaging device from the room view generator module; generate (208) at least one animated subject view (156) on the animated room view by inputting the at least one set of pose landmarks into the character mapping module; create (210) an animated image frame (158) by overlaying the at least one animated subject view on the animated room view; and assemble (212) the animated image frame into an anonymized video feed (160).

## Description

### FIELD OF THE INVENTION

The invention relates to medical imaging, in particular to the monitoring of medical imaging procedures.

### BACKGROUND OF THE INVENTION

Various tomographic medical imaging techniques such as Magnetic Resonance Imaging (MRI), Computed Tomography, Positron Emission Tomography, and Single Photon Emission Tomography enable detailed visualization of anatomical structure of a subject. A common feature of all of these imaging modalities is that these machines are complicated and require expertise and training to be able to use and/or repair them.

United States patent application publication US 2021/0353235 A1 discloses an avatar engine having a controller to retrieve a user profile of a user, present the user an avatar having characteristics that correlate to the user profile, detect one or more responses of the user during a communication exchange between the user and the avatar, identify from the one or more responses a need to determine a medical status of the user, establish communications with a medical diagnostic system, receive physiological information associated with the user, submit the physiological information to the medical diagnostic system, receive from the medical diagnostic system a diagnostic analysis of the physiological information, and present the diagnostic analysis to at least one of the user and a medical agent of the user, wherein the user is presented the diagnostic analysis by way of the avatar.

### SUMMARY OF THE INVENTION

The invention provides for a medical system, a method, and a computer program in the independent claims. Embodiments are given in the dependent claims.

As was mentioned above the use and maintaining of a medical imaging device such as a magnetic resonance imaging (MRI) system or a computed tomography system (CT) may require a significant experience to operate and maintain. The ability to monitor the use of a medical imaging device could be beneficial in improving the quality of care as well as reducing costs. A difficulty with such a strategy is that the privacy of subjects being imaged should be protected. In order to provide such privacy embodiments may provide a medical system that images the subject and displays an animation of the subject relative to the subject support of the medical imaging device. This may be used for providing detailed use of the medical imaging device in a confidential manner.

In one aspect the invention provides for a medical system that comprises a memory that stores machine-executable instructions, a body pose determination module, a character mapping module, and a room view generator module. The medical system further comprises a camera system. The camera system is configured for acquiring a video stream of a subject support of a medical imaging device within an examination room. A video stream as used herein encompasses a glimpse of images acquired by a camera system that are used to provide a video sequence or sequence of images. The video stream comprises individual image frames. The body pose determination module is configured to output a set of pose landmarks for at least one subject in response to receiving an individual frame of the individual imaging frames as input. In other words, when an individual frame is input into the body pose determination module a set of pose landmarks for at least one subject are output in response. The use of body pose determination modules is well known.

The pose landmarks could be joint locations. In some examples the pose landmarks may also include facial landmarks such as ear, nose etc. These facial landmarks could help in quantifying or displaying patient characteristics or moods such as anxiety or stress.

One well known example is the Kinect for the Xbox home entertainment system. Another well-known is the so-called MediaPipe Library, which is able to provide high-fidelity body pose trackers for inferring 33 three-dimensional landmarks and background segmentation masks for the whole body from an RGB video. The MediaPipe system for example, is able to run on current mobile phones, desktops within the Python programming language as well as being incorporated into websites.

The character mapping module is configured for providing an animated subject view by mapping an animated subject model onto the set of pose landmarks. For example, the character mapping module may be animation software that has an animated figure, in this case the animated subject model, which has its position specified by the same set of pose landmarks. The room view generator module is configured for generating an animated room view of the examination room and an animated view of the medical imaging device that is registered to the individual frame. This may for example be provided in a variety of ways. The room view generator module may take the video stream in some cases and use this for generating the room view generator module. In other cases, the animated room view may exist in advance and the individual frame is simply registered to this provided animated room view.

The room view generator module is further configured such that the animated view of the subject support is aligned with the use of the subject support in the individual image frames. This for example may be achieved in a variety of ways. In some cases there may be a sensor or sensors which detect the position of the subject support and this data is then used to adjust the animated room view. In other cases, an image segmentation algorithm may be used to segment the individual image and determine the position of the subject support and adjust the animated room view accordingly. For example, in one case, there may be a collection of animated room views with the subject support in different positions. Using sensor data or commands for positioning the subject support these same commands or data can then be used to select which of the animated room views is used to properly represent the position of the subject support.

The medical system further comprises a computational system. Execution of the machine-executable instructions causes the computational system to repeatedly control the camera system to acquire the video stream of the examination room. Execution of the machine-executable instructions further causes the computational system to repeatedly sequentially select the individual frame from the video stream. For each selected individual frame, execution of the machine-executable instructions causes the computational system to receive the set of pose landmarks for the at least one subject by inputting the individual frame into the body pose determination module. For each selected individual frame, execution of the machine-executable instructions causes the computational system to receive the animated room view of the medical imaging device from the room view generator module.

The animated room view is registered to the selected individual frame. For each selected individual frame, execution of the machine-executable instructions further causes the computational system to repeatedly generate at least one animated subject view on the animated room view by inputting the at least one set of pose landmarks into the character mapping module. The character mapping module receives the set of pose landmarks and then uses this to properly generate the at least one animated subject.

For each selected individual frame, execution of the machine-executable instructions further causes the computational system to create an animated image frame by overlaying the at least one animated subject view on the animated room view. For each selected individual frame, execution of the machine-executable instructions further causes the computational system to repeatedly assemble the animated image frame into an anonymized video feed.

This embodiment may be beneficial because it provides a means of depicting what is accurately occurring within the examination room without compromising any personal details of the subject. This for example, may improve the physical security of the examination room as well as provide a means of monitoring what is happening there and ensuring that the use of the medical imaging device is proceeding properly. The anonymized video feed for example, could be stored and maintained with any medical images obtained using the medical imaging device. This may be useful for example in providing more information on any obtained medical images for example to be used when the quality of the medical images is below a standard or a procedure needs to be repeated.

In another embodiment the memory further stores an activity classification module. The activity classification module is configured to output an activity classification in response to receiving the set of pose landmarks for the at least one subject as input. Execution of the machine-executable instructions further causes the computational system to repeatedly receive the activity classification in response to inputting the set of pose landmarks into the activity classification module and to append the activity classification to the anonymized video feed. The activity classification module may be used to identify what sort of activity or stage the subject is in. In the case of a medical imaging device, it may be useful for monitoring the pose or what stage the subject is in in preparation for execution of any medical imaging scan. Appending the activity classification to the anonymized video feed may also have the advantage in that the subject or people operating the camera system do not need to actively monitor the video feed in order to have a summary or report of what is occurring.

An activity detection module could be implemented in a variety of different ways. One could for example, define any by applying rules to the pose landmarks. If the pose landmarks satisfy predetermined conditions or orientation relative to each other, this could be used for generating the activity classification. Another way would be to look at a time series and use an LSTM neural network that looks at the poses as a function of time. For example, the neural network could take the various pose landmarks as input and then look at this as a time series and this may provide very accurate information as to what is occurring at a particular time in the examination room.

In another embodiment the memory further stores an object detection convolutional neural network that is configured to output an object identifier and object location for one or more objects selected from an object library in the individual frame. The object detection convolutional neural network may be any one of a number of standard neural network architectures that are used to identify and classify objects in an image. Examples of neural network architectures that may be useful may be an R-CNN neural network architecture or any one of the YOLO architectures. The object detection convolutional neural network may be trained by providing images from a video feed which have been labeled that label various objects from the object library. These labeled images may then be used for training the object detection convolutional neural network, for example, using a deep learning method.

Execution of the machine-executable instructions further causes the computational system to receive the object identifier and the object location if the one or more objects selected from the object library are detected in the individual frame. Execution of the machine-executable instructions further causes the computational system to overlay the one or more objects in the animated image frame by positioning them using the object location and the activity classification. This embodiment may be beneficial because it enables various objects to also be added to the anonymized video feed.

In another embodiment, execution of the machine-executable instructions further causes the computational system to receive an activity sequence defining a sequence of allowed activity classifications. Execution of the machine-executable instructions further causes the computational system to iteratively step through the activity sequence to determine if the activity classification deviates from the sequence of allowed activity classifications. Execution of the machine-executable instructions further causes the computational system to append a warning signal to the anonymized video feed if the activity classification deviates from the sequence of allowed activity classifications. This embodiment may be beneficial because it may provide for an automated means of informing the operator when the examination of the subject in the examination room is not proceeding as expected. This may also be useful in identifying when there are faults in using the medical imaging device.

In another embodiment the medical system further comprises a remote command center. The remote command center is configured for receiving the anonymized video feed via a network connection. The remote command center comprises a display configured for rendering the received anonymized video feed. This embodiment may be beneficial because it provides for a means of using the anonymized video feed to monitor the use of the medical imaging device in the examination room from a remote location. And it does this in a way where the security and the identity of the subject are not compromised.

In another embodiment the display is configured for receiving and displaying multiple anonymized video feeds. This may be beneficial because the remote command center may be used to monitor the operation of many different medical imaging devices at possibly very many different locations.

In another embodiment the display is further configured for modifying the display in the anonymized video feed if a warning signal is received. This may be useful because this may be used to automatically draw the attention of the operator within the remote command center to the anonymized video feed if there is a problem.

In another embodiment the remote command center is configured for sending commands or instructions to the computational system in response to receiving the anonymized video feed. This for example, may be commands or instructions which are provided to an operator of the medical imaging device. In other circumstances, this may be commands which are sent to a processor or computational system which is controlling the medical imaging device. This for example, may enable an operator in the remote command center to correct or assist in the procedure of imaging the subject with the medical imaging device. This may for example be useful in providing expertise which cannot be provided at every location economically.

In another embodiment the computational system is configured for receiving a subject support location signal from the medical imaging device. This for example, could be a position or coordinate of the subject support. In the case where the subject is loaded into the machine, such as for a CT, PET or MRI system, this may be used for identifying where or how far the subject is into the medical imaging device.

The room view generator is configured to receive the subject support location signal as input and adjust the animated room view in response. For example, the position of the subject support can be moved such that it reflects the subject support location signal. Execution of the machine-executable instructions further causes the computational system to receive the subject support location signal from the medical imaging device. Execution of the machine-executable instructions further causes the computational system to receive an updated animated room view in response to inputting the subject support location signal into the room view generator. The creation of the animated image frame is performed by overlaying the at least one animated subject view on the updated animated room view. This may for example be very useful in a situation where the subject has been placed on the subject support and then is in the process or has been loaded into the medical imaging device. This provides a means of making the animated room view more realistic and more reflect the actual configuration and use of the medical imaging device with the subject.

In another embodiment the anonymized video feed is assembled in real time. In this embodiment anonymizing the video feed in real time may encompass providing the anonymized video feed within a predetermined delay, for example, of maybe several seconds, a second or within several milliseconds. This may be beneficial because it may provide for an effective means of providing the actual situation in the examination room.

In another embodiment the medical system comprises the medical imaging device.

In another embodiment the medical imaging device is a magnetic resonance imaging system.

In another embodiment the medical imaging device is a magnetic resonance guided high-intensity focused ultrasound system.

In another embodiment the medical imaging device is a computed tomography system.

In another embodiment the medical imaging device is a digital X-ray system.

In another embodiment the medical imaging device is a digital fluoroscope.

In another embodiment the medical imaging device is a positron emission tomography system.

In another embodiment the medical imaging device is a single photon emission computed tomography system.

In another aspect the invention provides for a method of medical imaging. The method comprises controlling the camera system to acquire a video stream of an examination room. The camera system is configured for acquiring the video stream of a subject support of a medical imaging device within the examination room. The video stream comprises individual image frames. The method further comprises sequentially selecting the individual frame from the video stream.

The method further comprises for each selected individual frame, receiving a set of pose landmarks for the at least one subject by inputting the individual frame into a body pose determination module. The body pose determination module is configured to output a set of pose landmarks for at least one subject in response to receiving an individual frame of the individual image frames as input.

The method further comprises for each selected individual frame receiving an animated room view of the medical imaging device from a room view generator module. The room view generator module is further configured such that an animated view of the subject support is aligned with views of the subject support in the individual image frames.

The method further comprises for each selected individual frame generating at least one animated subject view on the animated room view by inputting the at least one set of pose landmarks into a character mapping module. The character mapping module is configured for providing an animated subject view by mapping an animated subject model onto the set of pose landmarks. The room view generator module is configured for generating an animated room view of the examination room and an animated view of the medical imaging device that is registered to the individual frame. The method further comprises for each selected individual frame, creating an animated image frame by overlaying the at least one animated subject view on the animated room view. The method further comprises for each selected individual frame, assembling the animated image frame into an anonymized video feed.

In another embodiment the method further comprises storing the anonymized video feed.

In another embodiment the method further comprises training a machine learning module with the anonymized video feed. Instead of training the machine learning module with the actual images of the subject being loaded into the medical imaging device, the anonymized video feed may be used instead. This may be beneficial because for example, it can be provided without the need of compromising the privacy of the subject.

During the training process feedback can be collected from users by replaying the anonymized video feed and the corresponding predictions from various artificial intelligence models used during this process. In the case of discrepancies, the users can correct the predictions. Such corrections may be used for retraining the artificial intelligence models. In other words, retrospective use of anonymized video feeds can be used for continuous learning of artificial intelligence models.

In another embodiment the method further comprises showing the anonymized video feed to an operator during training to operate the medical device. In this case, realistic scenarios can be stored and then shown to operators for training purposes.

In another embodiment the method further comprises displaying the anonymized video feed in real time at a remote location. For example, there may be a control center or centralized location where anonymized video feeds from many different devices may be displayed together.

In another embodiment the method further comprises showing the anonymized video feed to a subject prior to the use of the medical imaging device. This embodiment may be beneficial because realistic situations can be shown to a subject to explain to them what may happen during their own procedure. The use of the anonymized video feed may be beneficial because then the identity of people who have previously had the same procedure are not compromised.

In another aspect the invention provides for a computer program that comprises machine-executable instructions. The computer program may for example be stored on a non-transitory storage medium. The computer program comprises the machine-executable instructions as well as a body pose determination module, a character mapping module, and a room view generator module all for execution by a computational system. Execution of the machine-executable instructions causes the computational system to control a camera system to acquire a video stream of an examination room. The camera system is configured for acquiring the video stream of a subject support of a medical imaging device. The medical imaging device may be within an examination room. The video stream comprises individual image frames.

Execution of the machine-executable instructions further causes the computational system to sequentially select the individual frame from the video stream. Execution of the machine-executable instructions further causes the computational system, for each selected individual frame, to receive a set of pose landmarks for the at least one subject by inputting the individual frame into the body pose determination module. The body pose determination module is configured to output a set of pose landmarks for at least one subject in response to receiving the individual frame of the individual imaging frames as input. Execution of the machine-executable instructions further causes the computational system, for each selected individual frame, to receive the animated room view of the medical imaging device from the room view generator module.

The room view generator module is further configured such that an animated view of the subject support is aligned with views of the subject support in the individual image frames. Execution of the machine-executable instructions further causes the computational system, for each selected individual frame generated, to generate at least one animated subject view by inputting the at least one set of pose landmarks into the character mapping module. The character mapping module is configured for providing an animated subject view by mapping an animated subject model onto the set of pose landmarks. The room view generator module is configured for generating an animated room view of the examination room and an animated view of the medical imaging device that is registered to the individual frame.

Execution of the machine-executable instructions further causes the computational system, for each selected individual frame, to create an animated image frame by overlaying the at least one animated subject view on the animated room view. Execution of the machine-executable instructions further causes the computational system, for each selected individual frame, to assemble the animated image frame into an anonymized video feed.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Medical imaging data is defined herein as being recorded measurements made by a tomographic medical imaging system descriptive of a subject. The medical imaging data may be reconstructed into a medical image. A medical image is defined herein as being the reconstructed two- or three-dimensional visualization of anatomic data contained within the medical imaging data. This visualization can be performed using a computer.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance Imaging (MRI) image or MR image is defined herein as being the reconstructed two- or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 shows a flow chart which illustrates a method of using the medical system of Fig. 1;
Fig. 3 illustrates an example of a multiple anonymized video feeds;
Fig. 4 illustrates a further example of a medical system;
Fig. 5 illustrates how activity detection can be performed;
Fig. 6 illustrates the construction of an animated subject view; and
Fig. 7 illustrates the construction of an animated image frame.

### DESCRIPTION OF EMBODIMENTS

Like numbered elements in these figures are either equivalent elements or perform the same function. Elements which have been discussed previously will not necessarily be discussed in later figures if the function is equivalent.

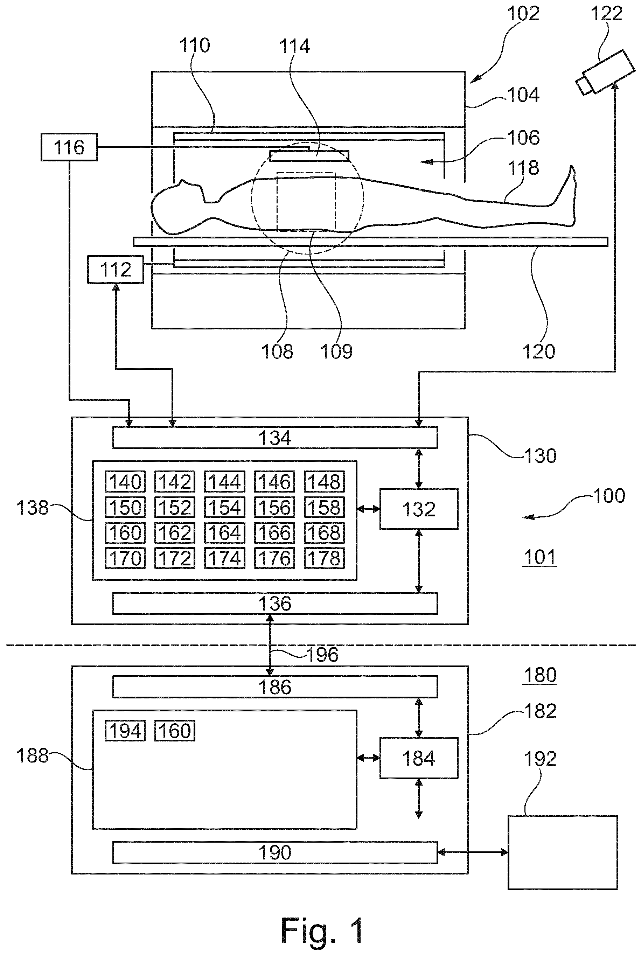

Fig. 1 illustrates an example of a medical system 100. The medical system 100 is located within an examination room 101. In this particular example, a magnetic resonance imaging system 102 is used as an example of a medical imaging device. However, other types of medical imaging devices, such as computed tomography systems, ultrasound systems or other medical scanners could be substituted in place of the magnetic resonance imaging system 102.

The magnetic resonance imaging system 102 comprises a magnet 104. The magnet 104 is a superconducting cylindrical type magnet with a bore 106 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet. An open magnet has two magnet sections, one above the other with a space in-between that is large enough to receive a subject. the arrangement of the two sections area similar to that of a Helmholtz coil. Open magnets are popular, because the subject is less confined. Inside the cryostat of the cylindrical magnet there is a collection of superconducting coils.

Within the bore 106 of the cylindrical magnet 104 there is an imaging zone 108 where the magnetic field is strong and uniform enough to perform magnetic resonance imaging. A field of view 109 is shown within the imaging zone 108. A subject support 120 supports a portion of a subject 118 in the imaging zone 108. The magnetic resonance data that is acquired typically acquried for the field of view 109.

Within the bore 106 of the magnet there is also a set of magnetic field gradient coils 110 which is used for acquisition of preliminary magnetic resonance data to spatially encode magnetic spins within the imaging zone 108 of the magnet 104. The magnetic field gradient coils 110 connected to a magnetic field gradient coil power supply 112. The magnetic field gradient coils 110 are intended to be representative. Typically magnetic field gradient coils 110 contain three separate sets of coils for spatially encoding in three orthogonal spatial directions. A magnetic field gradient power supply supplies current to the magnetic field gradient coils. The current supplied to the magnetic field gradient coils 110 is controlled as a function of time and may be ramped or pulsed.

Adjacent to the imaging zone 108 is a radio-frequency coil 114 for manipulating the orientations of magnetic spins within the imaging zone 108 and for receiving radio transmissions from spins also within the imaging zone 108. The radio frequency antenna may contain multiple coil elements. The radio frequency antenna may also be referred to as a channel or antenna. The radio-frequency coil 114 is connected to a radio frequency transceiver 116. The radio-frequency coil 114 and radio frequency transceiver 116 may be replaced by separate transmit and receive coils and a separate transmitter and receiver. It is understood that the radio-frequency coil 114 and the radio frequency transceiver 116 are representative. The radio-frequency coil 114 is intended to also represent a dedicated transmit antenna and a dedicated receive antenna. Likewise the transceiver 116 may also represent a separate transmitter and receivers. The radio-frequency coil 114 may also have multiple receive/transmit elements and the radio frequency transceiver 116 may have multiple receive/transmit channels.

The transceiver 116 and the gradient controller 112 are shown as being connected to the hardware interface 106 of the computer system 102. Both of these components, as well as others such as the subject support supplying positional data, may supply the sensor data 126.

The medical system 100 additionally comprises a camera system 122 that takes images within the examination room 101 such that at least the subject support 120 is imaged. The magnetic resonance imaging system 102 may or may not be part of the medical system 100.

The medical system 100 further comprises a computer 130 that has a computational system 132. The computer 130 is intended to represent one or more computer systems that may be located at the same location or networked together. Likewise, the computer 130 is shown as comprising a computational system 132 that may represent one or more computational cores. The computational system 132 is shown as being in connection with a hardware interface 134 that enables the computational system 132 to control and operate the magnetic resonance imaging system 102 or possibly another type of medical imaging device. The computational system 132 is further shown as being in connection with a network interface 136 and a memory 138. The network interface 136 enables the computational system 132 to communicate with other computer and computational systems. The memory 138 is intended to represent various types of memory or storage devices that may be in communication with the computational system 132. The memory 138 may be a non-transitory storage medium.

The memory 138 is shown as containing machine-executable instructions 140. The machine-executable instructions 140 enable the computational system 132 to perform various control and computational tasks. This may include such things as data and image processing. The memory 138 is further shown as containing a video stream 142 that has been acquired by the computational system 132 controlling the camera system 122. The memory 138 is further shown as containing an individual frame 144 that has been extracted from the video stream 142. Individual frames 144 may be extracted in sequence from the video stream 142.

The memory 138 is further shown as containing a body pose determination module 146. The memory 138 is further shown as containing a set of pose landmarks 148 that have been received from the body pose determination module 146 after inputting the individual frame 144 into it. The memory 138 is further shown as containing a room view generator module 150. The room view generator module 150 is configured to output an animated room view 152. The animated room view 152 may be provided in a variety of ways. For example, there could be a sensor or detector which detects the position of the subject support 120 and, depending upon the location of the subject support, a particular animated room view 152 is retrieved. In other examples the animated room view 152 may be generated from images of the video stream 142, where there are no subjects 118 present. In other examples, an image may be taken of the subject support 120 when there are no subjects present and the image may be put through an automated algorithm to turn the image into an animated image.

The memory 138 is further shown as containing a character mapping module 154. The character mapping module 154 may for example be animation software that has been configured to receive the set of pose landmarks 148. Upon receiving the set of pose landmarks 148 the character mapping module 154 generates an animated subject view 156. The animated subject view 156 is shown as being stored in the memory 138. The memory 138 is further shown as containing an animated image frame 158 that is generated by combining the animated subject view 156 with the animated room view 152. The memory 138 is then shown as containing an anonymized video feed 160 that has been generated by combining the animated image frame 158 as they are individually generated.

The memory 138 is further shown as optionally containing an activity classification module 162. The activity classification module 162 can for example take the set of pose landmarks 148 and use this to generate an activity. This may for example be done using a neural network or the relative position of the set of pose landmarks 148 may be used to classify the activity within the examination room 101. This for example could be a simple rule-based way of generating this activity classification. The memory 138 is further shown as containing an object detection convolutional neural network 164. The object detection convolutional neural network 164 is configured to output an object identifier 166 and an object location 168. This may be used for identifying various objects, for example in a predetermined library of objects, such as common items that might be found in an examination room like a wheelchair, a contrast agent injector or other equipment. In some examples, the activity classification or objects detected by the object detection convolutional neural network 164 may also be appended or added to the animated image frame 158.

The memory 138 is optionally containing an activity sequence. This for example, may be a list of various activity classifications within the examination room 101 during a particular procedure or imaging technique. The activity classification generated by the activity classification module 162 can be for example compared against this activity sequence 170 and it can be detected if the activity or sequence of activities is not what is expected. In this case an activity sequence warning signal 172 can be generated. This for example could be an optical or audio warning is provided to an operator of the magnetic resonance imaging system 102 as well as appending additional information to the anonymized video feed 160.

The memory 138 is shown as containing pulse sequence commands 174. The pulse sequence commands are commands or instructions which the computational system 132 can be used to control the magnetic resonance imaging system 102 to acquire k-space data 176 that is descriptive of the field of view 109. The memory 138 is shown as containing k-space data 176 that has been acquired by controlling the magnetic resonance imaging system 102 with the pulse sequence commands 174. The memory 138 is further shown as containing a magnetic resonance image 178 that has been reconstructed from the k-space data 176.

In the bottom half of Fig. 1 is a region 180 that represents an optional remote command center 180. There is a remote computer 182 that has a remote computational system 184, a remote network interface 186, a remote memory 188, and a remote user interface 190. The remote user interface 190 comprises a remote display. The network interface 136 and the remote network interface 186 form a network connection 196 that enable computer 130 and the remote computer 182 to exchange data and information. Within the remote memory 188 there are remote machine-executable instructions 194 that enable the remote computational system 184 to perform various data processing and computational tasks. The memory 188 is shown as further containing a copy of the anonymized video feed 160. This may be displayed on the remote display 192. This for example could enable technical experts or medical experts at a remote location to monitor the function and operation of the medical system 100. Because the anonymized video feed 160 has had the personal information of the subject 118 removed, there are no longer any privacy concerns.

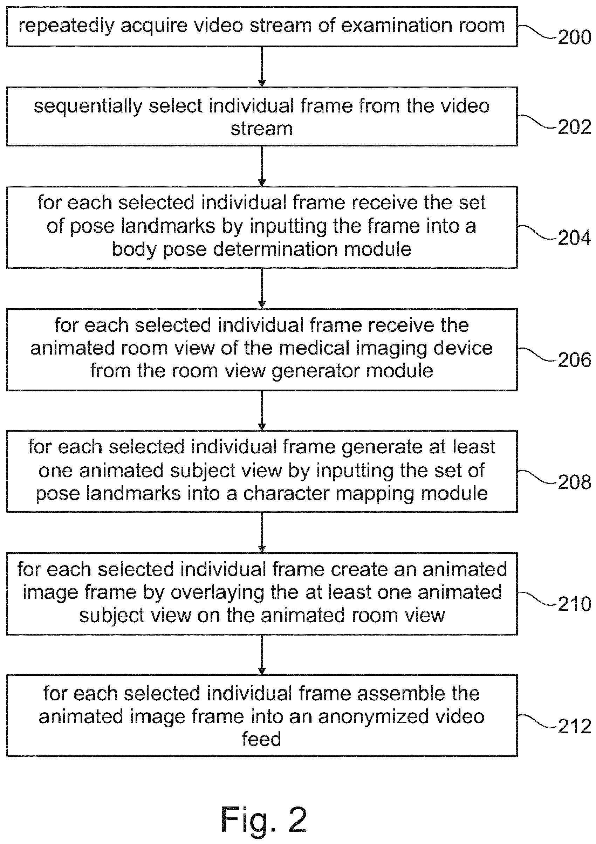

Fig. 2 shows a flowchart which illustrates one method of operating the medical system 100 of Fig. 1. First, in step 200, the camera system 122 is controlled to acquire the video stream 142 of the examination room 101. Next, in step 202, the individual frame 144 is sequentially selected from the video stream 142. Next, in step 204, for each selected individual frame 144 the set of pose landmarks 148 is received by inputting the individual frame 144 into the body pose determination module 146. Then, in step 206, for each selected individual frame 144 the animated room view 152 is received from the room view generator module 150. Then, in step 208, for each selected individual frame 144 at least one animated subject view 156 is received by inputting the at least one set of pose landmarks 148 into the character mapping module 154. Next, in step 210, for each selected individual frame 144 an animated image frame 158 is created by overlaying the at least one animated subject view 156 on the animated room view 152. Finally, for each selected individual frame 144 the anonymized video feed 160 is assembled from the animated image frame 158.

Although video feed from scanner room (examination room 101) is rich in clinical and operational insights, direct relay of this video on Radiology Operations Command Center (ROCC) (or remote command center 182) platform poses privacy-related concerns. Lack of this video feed (video stream 142) increases the cognitive load on the ROCC expert users to build the required contextual information while helping the local tech. Moreover, the use of these videos in training AI algorithms poses regulatory concerns. Examples may provide a system or enable a system that is aimed at using human pose (set of pose landmarks 148) to estimate the activity in an image acquisition workflow. Analysis of such derived activities provide novel clinical and operational insights. This human pose is in turn used to generate a live animated video that is relayed to ROCC users such as expert user. Such animated videos help in preserving patient and staff privacy.

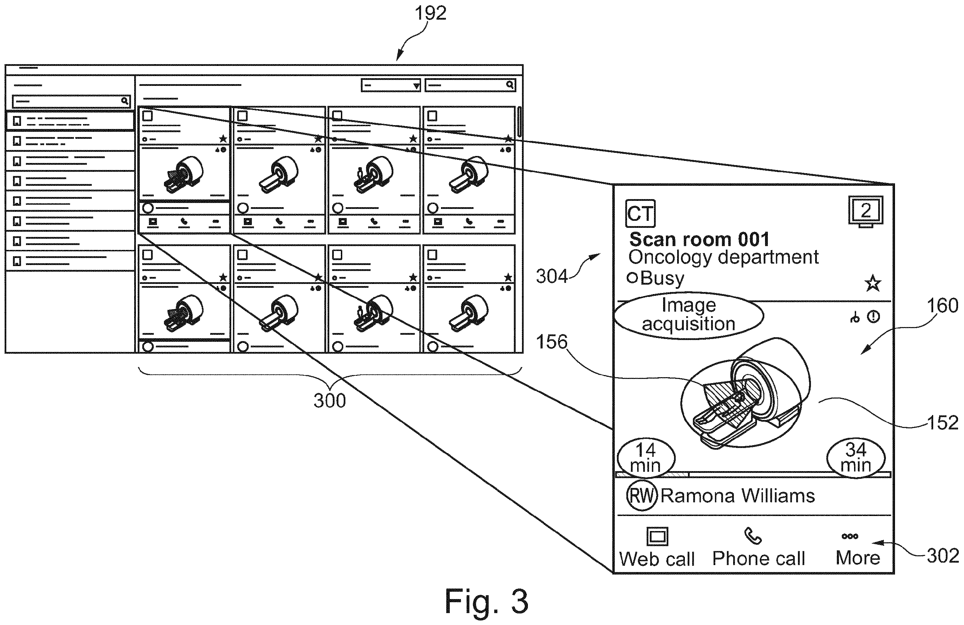

Radiology Operations Command Center (ROCC) offers the expert user to monitor several scanner rooms at a glance. It summarizes the key information onto an exam card to help expert user decide which room he/she needs to pay attention to. Note that the exam card (anonymized video feed 160) in Fig. 3 is currently displaying operational information derived from console screen. It is possible to extend this information to cover clinical aspects such as body part, contrast information, and patient position and other operational information derived from video feed captured by the camera.

Fig. 3 illustrates an example of a multiple anonymized video feeds 300. This for example may be displayed on the remote display 192. One of the multiple anonymized video feeds 300 is the anonymized video feed 160. The animated subject view 156 superimposed on the animated room view 152 is visible. In this case the anonymized video feed 160 additionally comprises a number of communication controls 302 that may enable the operator of the remote command center 180 to communicate with the operator of the medical system 100 via the network connection 196.

The communication controls 302 may also enable communication via other communication systems such as the telephone system. The anonymized video feed 160 may in its user interface also contain other information such as location and status information 304.

ROCC has a feature of relaying the video feed from a camera mounted in the local tech's scanner/control room (with a view of the examination room 101). This video feed enables the remotely located expert user get a better understanding of the situation that the local tech is in and thereby help in troubleshooting. This video feed may contain face and body of different individuals including patient and staff. There is a need to protect the privacy of the individuals involved in this video. This privacy preservation is especially important for patients as some imaging exams may require patients to expose certain body parts while being scanned.

In ROCC, an expert user monitors multiple imaging devices or scanners (MR, CT etc.) at the same time. In order to help the expert user to identify the scanner that they need to pay attention to, ROCC provides alerts (warning signals 408) to the exam card. In turn, these alerts are based on the clinical and operational situation of each imaging scanner. Currently, the operational situation is primarily derived from information captured on the console screen. However, the video feed from camera has some rich operational information such as patient on/off table, patient in a wheelchair. In some imaging centers, as a practice to preserve patient privacy or comply with the privacy law of the land, local tech may close the blinds on the scanner room window facing the control room or stop the video feed entirely. These practices inhibit ROCC from capturing this rich operational information from the video feed. In this invention disclosure we provide a system that enables ROCC to derive the required operational information from the video feed while preserving the privacy of all the individuals in the video feed.

Additionally, in order to allow sites to expand the pool of potential expert users (i.e. borrow experts from other organizations), ROCC may create a level of abstraction between restricted, privacy-protected information/views and experts that may not have complete organizational privileges.

Apart from deriving the operational metrics from the video feed, expert users also want to get a sense of the situation in the control/scanner room. This could be about the coils available in the scanner room, scanner room layout etc. This is especially important as expert users deal with multiple scanners. However, direct relay of the live video feed from the scanner room poses concerns around patient/staff privacy. This invention disclosure addresses this problem by converting the live video feed into animated video feed.

Patients often face anxiety when they are scheduled for an imaging exam. Currently, patients are referred to some online videos on what to expect. However, these videos are idealistic in nature and lack the information on what happens at the imaging center that their exam is scheduled. This invention disclosure addresses this problem by providing the imaging center an animated video of what can happen in each of the scanner rooms for various exams. The imaging center can use such videos with their patients and help them educate and prepare for their exam. Such videos may also be used for new hire trainings - protecting patient/staff privacy, yet clearly modelling correct behaviors, illustrating challenging patient behaviors, errors, etc.

Examples may include one or more of the following features:
- Module 1: A module to extract the images from video feed from camera
- Module 2: A module to detect the human pose and its associated activity (optionally: detect other movable objects and their position in space)
- Module 3: A module to derive animated/movable objects based on the pose detected in Module 2
- Module 4: A module to create animated video based on animated/movable objects from Module 3
- Module 5: A module to present operational and clinical insights from pose and activity detected in Module 2

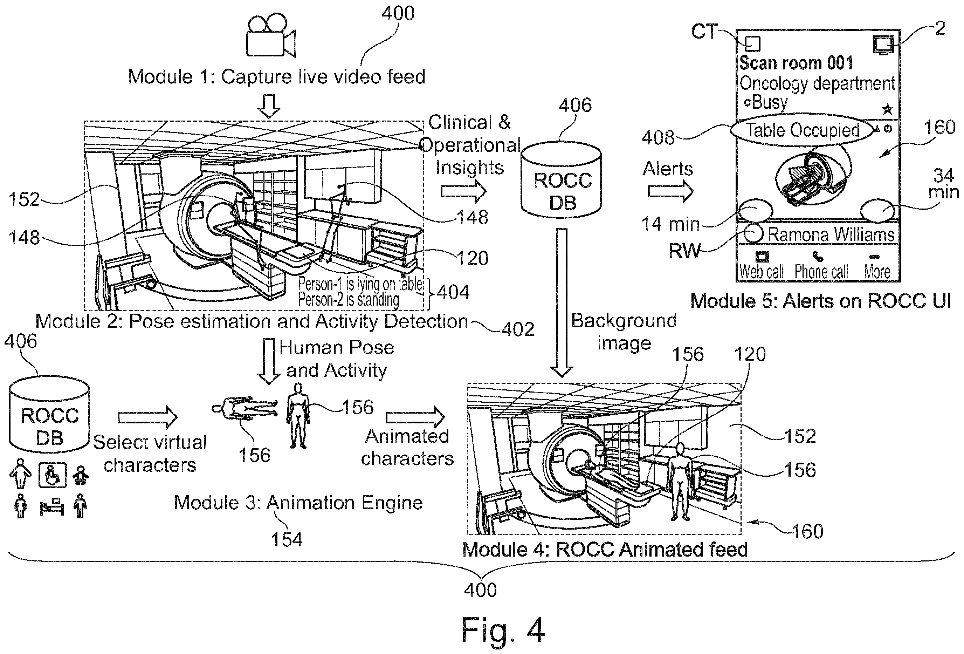

Fig. 4 illustrates a further example of a medical system 400. Each module in this figure is described in detail in this section. The medical system 400 in Fig. 4 is illustrated in a functional manner. A first module represents the capturing of the live video feed 400. A second module, 402, represents a post estimation and optionally activity detection. There is an example animated room view 152, which shows two separate sets of pose landmarks 148. In this example the animated room view 152 is a view of the examination room 101 without any subjects in the room. Superimposed on the image 152 are activity classifications 404 for the two subjects. One is lying on the table and the other is standing.

There is optionally a database 406 which may be used for a variety of functions or may represent multiple databases. In one case the database 406 is used in conjunction with the character mapping module 154 or animation engine. For example, the database 406 can be used to select virtual characters which may be used for providing characters for the animated subject view 156. The set of pose landmarks 148 is input to this animation engine 154 as well as the detected locations of any objects in the video feed also. This then results in animated characters which can be superimposed on the animated room view 152 to provide an anonymized video feed 160. In this example, the anonymized video feed 160 shows two subjects 156 within the animated room view 152.

The database 406 may also be used to provide the animated room view 152. For example, the position of the subject support 120 may be used to recall a prerecorded image which may be used as the animated room view 152. The database 406 may also contain various rules or artificial intelligence modules which can be used to provide alerts 408 which may be displayed on the anonymized video feed 160.

Various (executable) modules may also be included:
1. A module for parsing the live video feed from camera into images: This module converts the live video feed from camera into images.
2. A module to detect the human pose and its associated activity, and other movable objects: This module has a stack of algorithms such as object detection, object tracking to identify the object in a given image. This detected object is further classified into a person or non-living object. A pose estimation algorithm is run on the detected persons in the image and keypoints such as joints for each detected person are identified. These keypoints are further fed into a classification algorithm to classify the pose into certain human actions such as sitting, lying on table, standing etc. In addition to identifying human pose, the module may optionally also detect other movable objects of specific interest, such as the position of a movable patient table, the position of MRI coils, the position of a contrast injector, or other devices.

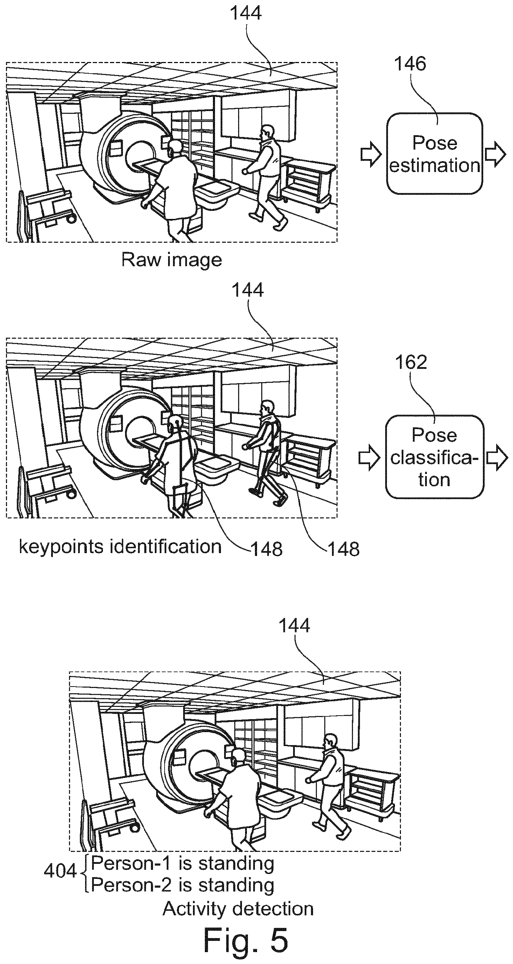

Fig. 5 illustrates how activity detection can be performed using keypoints or pose landmarks 148. The first image 144 is an individual frame and represents a raw image. This may then be used in some form of pose estimation. In examples this may be done using the body pose determination module 146. The body pose determination module 146 then outputs a set of pose landmarks 148 which are shown as being represented on the individual frame 144. The set of pose landmarks 148 is equivalent to key point identification. Once the sets of pose landmarks 148 have been determined, these may then be input into a pose classification or an activity classification module 162. Either an artificial intelligence module may look at the evolution in time of the various coordinates for subjects or, for example, a rule-based system may be used. The activity classification module 162 then outputs a number of activity classifications 404.

This module can use off-the-shelf AI models such as mediapipe, openpose, alphapose for estimating the pose. Alternatively, there are human activity detection models such as slowfast that directly detect the activity. In ROCC, the computational source available to run these AI models is a surface pro tablet. The selection of these algorithms is tricky for the following two reasons: 1) The results from the stack of these algorithms need to be in real time and, 2) ROCC tablets offer a limited computational power.

3. A module to derive animated objects based on the pose/activity detected in Module 2: It is assumed that there is a set of animated characters available on ROCC database. Before beginning of an exam, animated characters are selected to represent different persons including staff and patient. It is possible to hide patient characteristics including gender, BMI of the patient by choosing the virtual character appropriately. This choice can be made by patient or local tech or a random selection by the software.

These animated characters may provide the surface representation of the animated object and the keypoints, pose and activity provide the skeletal representation of the animated object. All this information about the animated object is rigged into an animated object for a given image. There are multiple standard softwares such as Mixamo that provide this functionality of superimposing the skeletal pose onto a virtual character.

Other movable objects, such as a patient table, MRI coils, or other equipment, can also be represented by 3D models from model libraries.

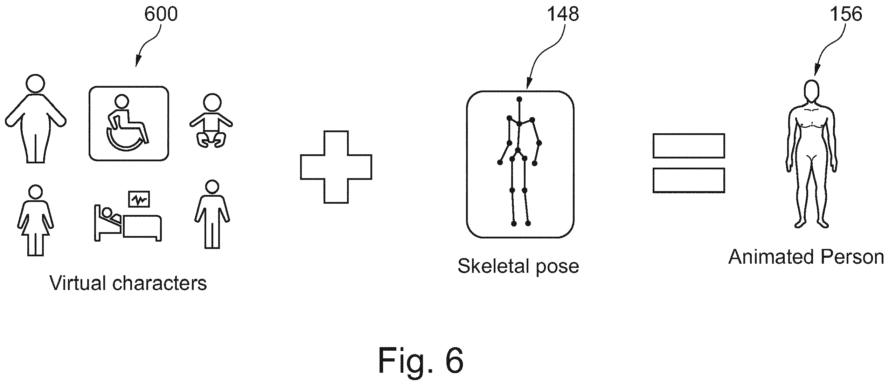

Fig. 6 illustrates the construction of an animated subject view 156. In this example, there are a number of virtual characters 600 which may be selected. The character mapping module 154 or animation module takes one of these virtual characters 600 and a skeletal pose or set of pose landmarks and then uses this to output or generate the animated subject view 156. In this case it represents an animated person.

4. A module to create animated video based on animated objects from Module 3: This module receives a continuous feed of animated objects from module 3. These objects are overlayed onto a background image to generate a video feed. Note that this video feed does not contain the real person's face or body and there by this animated video feed can be relayed to expert user. In addition to the overlay of the animated person, other detected movable objects can also be overlayed on the image at their respective locations in space. In this way, the observer would immediately see that, e.g., a flexible MRI coil has been placed on the patient's body.

The position of the MRI or CT table/couch can lead to unrealistic representations of the animated patient figure. If a patient is moved into the bore, the animated figure would be moving while the patient table on the background image remains static. To solve this problem, the patient table could also be represented in a different way: Instead of using just one reference image for the background, a series of images with different patient table positions is recorded. The location of the patient table detected by the object detection algorithm in Module 2 is then used to select the background image that matches the real patient table position best. In this way, a patient being moved in and out of the MRI bore would be displayed as an animated figure on a (stepwise) "moving" patient table.

There are multiple use cases for the animated video generated by this Module such as
1. It can be used to retrain the AI models described in Module 2 for continuous improvement.
2. It can be used for patient education. A video corresponding to patient's exam can help them learn key instructions such as how to lie on the patient table, breathing instructions and thereby reduce anxiety on what to expect during the exam. In the same spirit, these animated videos can be used for staff education, especially for novice technologists, as well.

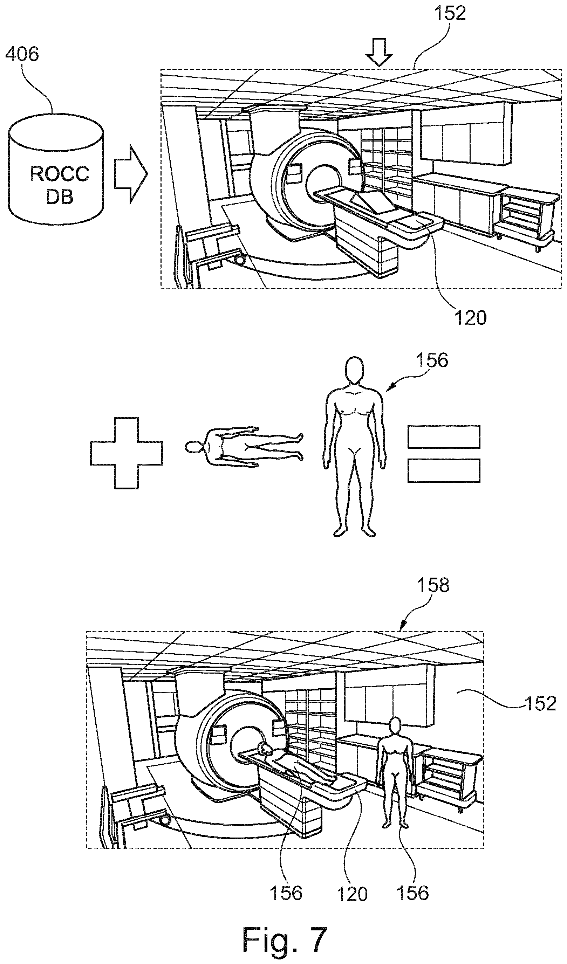

Fig. 7 illustrates the construction of an animated image frame 158. The database 406 is used to select an animated room view 152 based on a particular subject support 120 position. In this case the animated room view 152 is a real-life image that was acquired when no subjects or people were in the examination room. In the next step various views of the animated subject view 156 are provided such as was illustrated in Fig. 6. The animated subject view 156 is then superimposed on the animated room view 152 to provide an animated image frame 158. These frames 158, as they are generated, can be combined into an anonymized video feed 160.

5. A module to capture operational and clinical information using pose and activity detected in Module 2: Pose and activity from Module 2 reveal novel operational and clinical insights about patient and status of the imaging exam. This information can be used to create alerts on the ROCC UI in various contexts (see Figure 6 for more details). A few examples are discussed below:
a. Pose can be used in estimating patient's mobility. For instance, patient arrived for imagining exam in a wheelchair/gurney/walked-in is a good indicator of patient mobility. This detection of wheelchair or gurney can be performed by using object detection algorithms such as detectron2, Yolo etc. Alert can be triggered to expert tech so that he/she work with transport department at the hospital while local tech is busy scanning the patient.
b. Based on the pose and activity of different persons in the scanner room, it is possible to classify the image acquisition workflow into different steps such as table occupied, patient positioning, cleaning, scanning etc. This classification could be performed using rule-based techniques or using advanced AI techniques such as LSTM. Timeseries analysis of these workflow steps can provide insights into the delays. Unusual delays are notified to expert techs via alerts.
c. Pose can further be used to create patient safety alerts. The position of head with respect to the scanner bore help us in estimating the patient position as well. This patient positioning is pre-determined based on the nature of patient's imaging exam. An alert to expert tech can be triggered if local tech is incorrectly positioning the patient.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 101: examination room
- 102: magnetic resonance imaging system
- 104: magnet
- 106: bore of magnet
- 108: imaging zone
- 109: field of view
- 110: magnetic field gradient coils
- 112: magnetic field gradient coil power supply
- 114: radio-frequency coil
- 116: transceiver
- 118: subject
- 120: subject support
- 122: camera system
- 130: computer
- 132: computational system
- 134: hardware interface
- 136: network interface
- 138: memory
- 140: machine executable instructions
- 142: video stream
- 144: individual frame
- 146: body pose determination module
- 148: set of pose landmarks
- 150: room view generator module
- 152: animated room view
- 154: character mapping module
- 156: animated subject view
- 158: animated image frame
- 160: anonymized video feed
- 162: activity classification module
- 164: object detection convolutional neural network
- 166: object identifier
- 168: object location
- 170: activity sequence
- 172: activity sequence warning signal
- 174: pulse sequence commands
- 176: k-space data
- 178: magnetic resonance image
- 180: remote command center
- 182: remote computer
- 184: remote computational system
- 186: remote network interface
- 188: remote memory
- 190: remote user interface
- 192: remote display
- 194: remote machine executable instructions
- 196: network connection
- 200: control the camera system to acquire the video stream of the examination room
- 202: sequentially select the individual frame from the video stream
- 204: for each selected individual frame receive the set of pose landmarks for the at least one subject by inputting the individual frame into the body pose determination module
- 206: for each selected individual frame receive the animated room view of the of the medical imaging device from the room view generator module
- 208: for each selected individual frame generate at least one animated subject view on the animated room view by inputting the at least one set of pose landmarks into the character mapping module
- 210: for each selected individual frame create an animated image frame by overlaying the at least one animated subject view on the animated room view
- 212: for each selected individual frame assemble the animated image frame into an anonymized video feed
- 300: multiple anonymized video feed
- 302: communication controls
- 304: location and status information
- 400: capture live video feed
- 402: pose estimation and activity detection
- 404: activity classifications
- 406: database
- 408: warning signal (alert)
- 600: selection of virtual characters

## Claims

1. A medical system (100, 400) comprising:
a memory (138) storing machine executable instructions (140), a body pose determination module (146), a character mapping module (154), and a room view generator module (150);
a camera system (122), wherein the camera system is configured for acquiring a video stream (142) of a subject support (120) of a medical imaging device (102) within an examination room (101), wherein the video stream comprises individual image frames, wherein the body pose determination module is configured to output a set of pose landmarks (148) for at least one subject in response to receiving an individual frame of the individual imaging frames as input, wherein the character mapping module is configured for providing an animated subject view (156) by mapping an animated subject model (600) onto the set of pose landmarks, wherein the room view generator module is configured for generating an animated room view (152) of the examination room and an animated view of the medical imaging device that is registered to the individual frame, wherein the room view generator module is further configured such that an animated view of the subject support is aligned with views of the subject support in the individual image frames; and
a computational system (132), wherein execution of the machine executable instructions causes the computational system to repeatedly:
control (200) the camera system to acquire the video stream of the examination room;
sequentially (202) select the individual frame from the video stream;
for each selected individual frame receive (204) the set of pose landmarks for the at least one subject by inputting the individual frame into the body pose determination module;
for each selected individual frame receive (206) the animated room view of the of the medical imaging device from the room view generator module, wherein the animated room view is registered to the selected individual frame;
for each selected individual frame generate (208) at least one animated subject view (156) on the animated room view by inputting the at least one set of pose landmarks into the character mapping module;
for each selected individual frame create (210) an animated image frame (158) by overlaying the at least one animated subject view on the animated room view; and
for each selected individual frame assemble (212) the animated image frame into an anonymized video feed (160).

2. The medical system of claim 1, wherein the memory further stores an activity classification module (162), wherein the activity classification module is configured to output an activity classification (404) in response to receiving the set of pose landmarks for the at least one subject as input, wherein execution of the machine executable instructions further causes the computational system to repeatedly:
receive the activity classification in response to inputting the set of pose landmarks into the activity classification module; and
append the activity classification to the anonymized video feed.

3. The medical system of claim 2, wherein the memory further stores an object detection convolutional neural network (164) configured to output an object identifier and object location for one or more objects selected from an object library in the individual frame; wherein execution of the machine executable instructions further causes the computational system to:
receive the object identifier and the object location if the one or more objects selected from the object library are detected in the individual frame; and
overlay the one or more objects in the animated image frame by positioning them using the object location and the activity classification.

4. The medical system of claim 2 or 3, wherein execution of the machine executable instructions further causes the computational system to:
receive an activity sequence defining a sequence of allowed activity classifications;
iteratively step through the activity sequence to determine if the activity classification deviates from the sequence of allowed activity classifications; and
append a warning signal (408) to the anonymized video feed if the activity classification deviates from the sequence of allowed activity classifications.

5. The medical system of any one of the preceding claims, wherein the medical system further comprises a remote command center (180), wherein the remote command center is configured for receiving the anonymized video feed via a network connection (196), wherein the remote command center comprises a display configured for rendering the received anonymized video feed.

6. The medical system of claim 5, wherein the display is configured for receiving and displaying multiple anonymized video feeds (300).

7. The medical system of claim 5 or 6, the display is further configured for modifying the display of the anonymized video feed if a warning signal is received.

8. The medical system of claim 5, 6, or 7, wherein the remote command center is configured for sending commands or instructions to the computational system in response to receiving the anonymized video feed.

9. The medical system of any one of the preceding claims, wherein the computational system is configured for receiving a subject support location signal from the medical imaging device, wherein the room view generator module is configured to receive the subject support location signal as input and adjust the animated room view in response, wherein execution of the machine executable instruction further causes the computational system to:
receive the subject support location signal from the medical imaging device; and
receive an updated animated room view in response to inputting the subject support location signal into the room view generator, wherein the creation of the animated image frame is performed by overlaying the at least one animated subject view on the updated animated room view.

10. The medical system of any one of the preceding claims, wherein the anonymized video feed is reassembled in real time.

11. The medical system of any one of the preceding claims, wherein the medical system comprises the medical imaging device.

12. The medical system of any one of the preceding claims, wherein the medical imaging device is any one of the following: a magnetic resonance imaging system (102), a magnetic resonance guided high intensity focused ultrasound system, a computed tomography system, a digital x-ray system, a digital fluoroscope, a positron emission tomography system, and a single photon emission computed tomography system.

13. A method of medical imaging, wherein the method comprises:
controlling (200) a camera system (122) to acquire a video stream (142) of an examination room (101), wherein the camera system is configured for acquiring the video stream of a subject support (120) of a medical imaging device (102) within the examination room, wherein the video stream comprises individual image frames;
sequentially (202) selecting an individual frame (144) from the video stream;
for each selected individual frame receiving (204) a set of pose landmarks (148) for the at least one subject by inputting the individual frame into a body pose determination module (146), wherein the body pose determination module is configured to output the set of pose landmarks for at least one subject in response to receiving an individual frame of the individual imaging frames as input; for each selected individual frame receiving (206) an animated room view (152) of the of the medical imaging device from a room view generator module, wherein the room view generator module is further configured such that an animated view of the subject support is aligned with views of the subject support in the individual image frames;
for each selected individual frame generating (208) at least one animated subject view on the animated room view by inputting the at least one set of pose landmarks into a character mapping module (154), wherein the character mapping module is configured for providing the animated subject view by mapping an animated subject model (600) onto the set of pose landmarks, wherein the room view generator module is configured for generating an animated room view (152) of the examination room and an animated view of the medical imaging device that is registered to the individual frame;
for each selected individual frame creating (210) an animated image frame by overlaying the at least one animated subject view on the animated room view; and
for each selected individual frame assembling (212) the animated image frame into an anonymized video feed.

14. The method of claim 13, wherein the method further comprises any one of the following:
storing the anonymized video feed;
training a machine learning module with the anonymized video feed;
showing the anonymized video feed to an operator during training to operate the medical device;
displaying the anonymized video feed in real time at a remote location;
showing the anonymized video feed to a subject prior to use of the medical imaging device to image the subject; and
combinations thereof.

15. A computer program comprising machine executable instructions, a body pose determination module, a character mapping module, and a room view generator module for execution by a computational system; wherein execution of the machine executable instructions causes the computational system to:
Control (200) a camera system (122) to acquire a video stream (142) of an examination room (101), wherein the camera system is configured for acquiring the video stream of a subject support (120) of a medical imaging device (102) within an examination room, wherein the video stream comprises individual image frames;
sequentially (202) select an individual frame (144) from the video stream;
for each selected individual frame receive (204) a set of pose landmarks (148) for the at least one subject by inputting the individual frame into a body pose determination module (146), wherein the body pose determination module is configured to output a set of pose landmarks for at least one subject in response to receiving the individual frame of the individual imaging frames as input;
for each selected individual frame receive (206) an animated room view (152) of the of the medical imaging device from the room view generator module, wherein the room view generator module is further configured such that an animated view of the subject support is aligned with views of the subject support in the individual image frames;
for each selected individual frame generate (208) at least one animated subject view on the animated room view by inputting the at least one set of pose landmarks into the character mapping module (154), wherein the character mapping module is configured for providing an animated subject view by mapping an animated subject model (600) onto the set of pose landmarks, wherein the room view generator module is configured for generating an animated room view (152) of the examination room and an animated view of the medical imaging device that is registered to the individual frame;
for each selected individual frame create (210) an animated image frame by overlaying the at least one animated subject view on the animated room view; and
for each selected individual frame assemble (212) the animated image frame into an anonymized video feed.
